# EUROPEAN PATENT SPECIFICATION

(11) **EP 0 660 714 B1**
(45) Date of publication and mention of the grant of the patent: **02.07.2003**
(21) Application number: 92912662.1
(22) Date of filing: 31.03.1992
(51) Int. Cl.: A61K 9/127

(54) **NOVEL LIPOSOMAL DRUG DELIVERY SYSTEMS**
NEUE LIPOSOMALE ARZNEIMITTELFREISETZUNGSSYSTEME
NOUVEAUX SYSTEMES D'APPORT DE MEDICAMENTS AUX LIPOSOMES

(30) Priority: 18.06.1991 US 716899
(43) Date of publication of application: 05.07.1995
(73) Proprietor: ImaRx Pharmaceutical Corp., Tucson, AZ 85719 (US)
(72) Inventor: Unger, Evan C., Tucson, Arizona 85749 (US); Wu, Guanli, Tucson, Arizona 85745 (US)
(74) Representative: Hallybone, Huw George
(86) International application number: US9202614
(87) International publication number: WO92022298

(56) References cited:
- EP-A- 0 243 947
- WO-A-86/03938
- WO-A-90/00389
- DE-A- 2 818 655
- DE-A- 4 013 580
- US-A- 4 657 756
- US-A- 4 675 310
- US-A- 4 900 540

## Description

This invention relates to the field of drug delivery and more specifically, to gas filled liposomes prepared using vacuum drying gas instillation methods, and to gas filled liposomes at least 90% devoid of liquid in the interior thereof, said liposomes also having encapsulated therein a drug. The invention further relates to methods of preparing such drug containing gas filled liposomes and to methods for employing such liposomes as drug delivery systems.

For hundreds of years man has endeavoured to develop a wide variety of drugs useful in the treatment of various diseases and disorders. Lagging noticeably behind in this effort, however, has been the development of effective means to deliver such drugs to selected sites in the body. Although some achievements have been made in this area during the last decade, see, e.g., R. Baker, Controlled Release of Biologically Active Agents, John Wiley & Sons (New York 1987), new and/or better drug delivery systems are needed.

Targeted delivery means are particularly important where drug toxicity is an issue. Specific drug delivery methods potentially serve to minimize toxic side effects, lower the required dosage amounts, and decrease drug costs for the patient. The present invention is directed to addressing these and/or other important needs in the area of drug delivery.

US-A-4675310 describes the use of ligands and phospholipid liposomes as a carrier for the transport of various gases. For example, the ligand may be hemoglobin entrapped in the liposome, for the transport of oxygen.

US-A-4900540 describes phospholipid liposomes entrapping a composition which includes a gas or gas precursor. The liposomes are useful as ultrasonic imaging contrast agents.

The present invention provides a drug delivery system comprising gas filled liposomes at least 90% devoid of liquid in the interior thereof and having encapsulated therein a drug, said liposomes being prepared by a vacuum drying gas instillation method comprising the steps of:
(i) applying negative pressure to a vessel containing liposomes having encapsulated therein said drug;
(ii) incubating said liposomes under said negative pressure, at a temperature above 0°C, for a time sufficient to remove at least 90% of the liquid from said liposomes; and
(iii) instilling gas into said liposomes until ambient pressures are achieved, to produce gas-filled liposomes which contain said drug.

The present invention also provides a drug delivery system comprising echogenic, gas-filled liposomes at least 90% devoid of liquid in the interior thereof having a gas and a drug encapsulated therein, wherein said liposomes are capable of being induced to rupture or release said drug upon the application of ultrasound.
The present invention also provides a method for preparing a drug delivery system comprising the following steps:
(i) applying negative pressure to a vessel containing liposomes having encapsulated therein a drug;
(ii) incubating said liposomes under said negative pressure, at a temperature above 0°C, for a time sufficient to remove at least 90% of the liquid from said liposomes; and
(iii) instilling gas into said liposomes until ambient pressures are achieved, to produce gas-filled liposomes which contain said drug.

The present invention also provides a method for preparing drug delivery systems for use in conjunction with ultrasonic imaging comprising the following steps:
(i) allowing liposomes having encapsulated therein a drug to cool to a temperature between -10°C and -20°C;
(ii) placing said liposomes in a vessel under a negative pressure of between 700 mm Hg to 760 mm Hg;
(iii) allowing said liposomes to warm to a temperature of between 10°C and 20°C;
(iv) incubating said liposomes under said negative pressure for 24 to 72 hours to remove at least 90% of the liquid from said liposomes; and
(v) instilling gas into said liposomes over a period of 4 to 8 hours until ambient pressures are achieved, while allowing said liposomes to warm to ambient temperature.
allowing them to be monitored effectively *in vivo*. The liposomes of the invention are also susceptible to rupture upon application of ultrasound at the peak resonant frequency of the liposomes. Such characteristics permit the subject liposomes to be employed in the novel and unexpected applications of the present invention. Further, the subject liposomes are surprisingly highly stable to pressure and/or possess a long storage life, either when *stored* dry or suspended in a liquid medium. Also unexpected is the ability of the liposomes during the vacuum drying gas instillation process to fill with gas and resume their original circular shape, rather than irreversibly collapse into a cup-like shape.

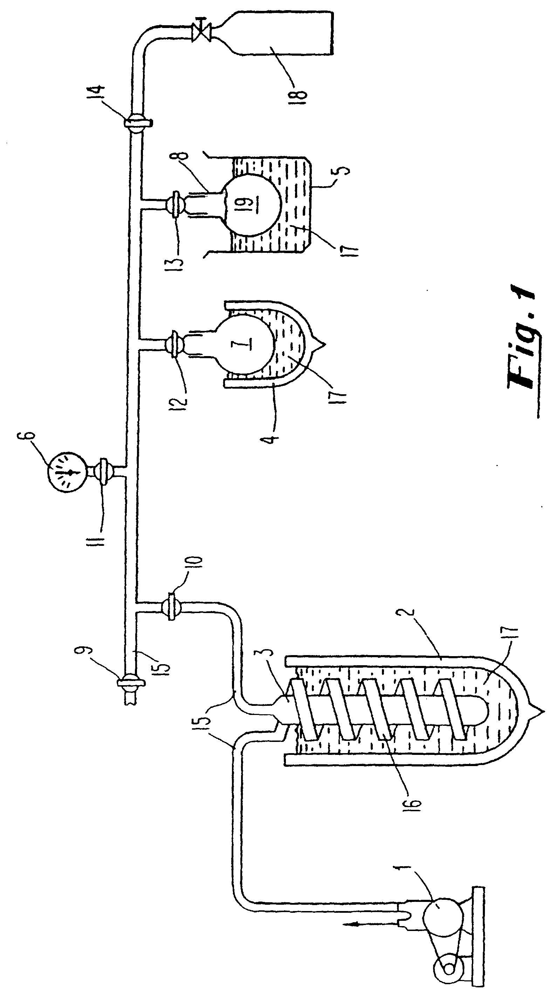

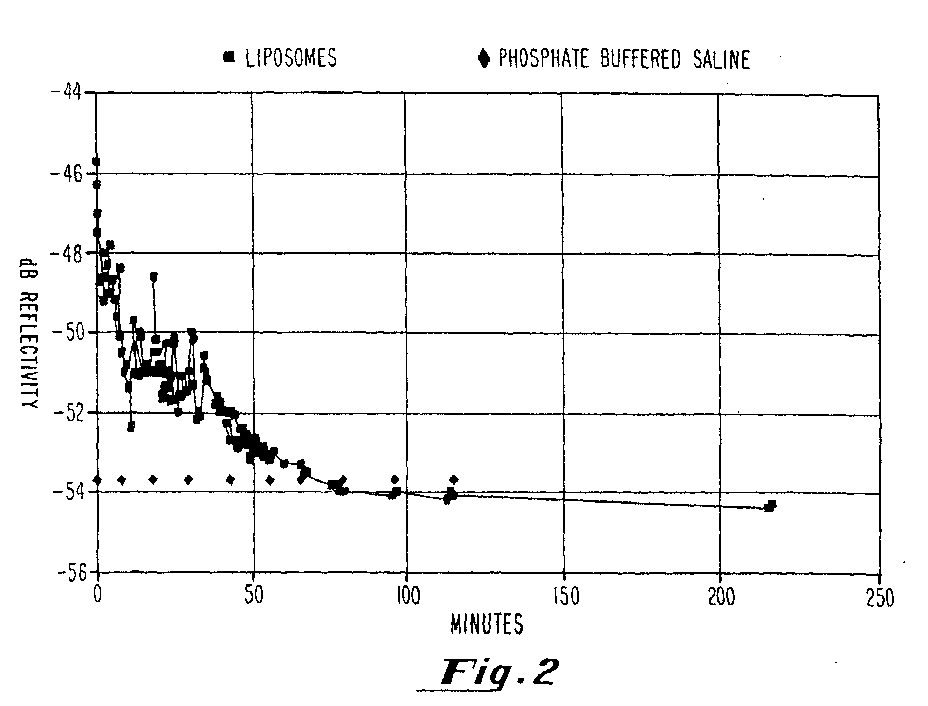

These and other features of the invention and the advantages thereof will be set forth in greater detail in the figures and the description below. In the figures:-
FIGURE 1 shows an apparatus for preparing the drug containing vacuum dried gas instilled liposomes, and the drug containing gas filled liposomes substantially devoid of liquid in the interior thereof prepared by the vacuum drying gas instillation method. FIGURE 2 is a graphical representation of the dB reflectivity of gas filled liposomes substantially devoid of liquid in the interior thereof prepared by the vacuum drying gas instillation method, without any drugs encapsulated therein. The data was obtained by scanning with a 7.5 megahertz transducer using an Acoustic Imaging™ Model 5200 scanner (Acoustic Imaging, Phoenix, Arizona), and was generated by using the system test software to measure reflectivity. The system was standardized prior to each experiment with a phantom of known acoustic impedance.

The present invention is directed to ultrasound contrast agents comprising gas filled liposomes prepared by vacuum drying gas instillation methods and having encapsulated therein a drug (that is, drug containing), such liposomes sometimes being referred to herein as drug containing vacuum dried gas instilled liposomes. The present invention is further directed to contrast agents comprising drug containing gas filled liposomes substantially devoid of liquid in the interior thereof.

The vacuum drying gas instillation method employed to prepare both the subject gas filled liposomes prepared by the vacuum drying gas instillation method, and the gas filled liposomes substantially devoid of liquid in the interior thereof, contemplates the following process. First, in accordance with the process, the drug containing liposomes are placed under negative pressure (that is, reduced pressure or vacuum conditions). Next, the liposomes are incubated under that negative pressure for a time sufficient to remove substantially all liquid from the liposomes, thereby resulting in substantially dried liposomes. By removal of substantially all liquid, and by substantially dried liposomes, as those phrases are used herein, it is meant that the liposomes are at least 90% devoid of liquid, preferably at least 95% devoid of liquid, most preferably about 100% devoid of liquid. Although the liquid is removed, the drug, with its higher molecular weight, remains behind, encapsulated in the liposome. Finally, the liposomes are instilled with selected gas by applying the gas to the liposomes until ambient pressures are achieved, thus resulting in the subject drug containing vacuum dried gas instilled liposomes of the present invention, and the drug containing gas filled liposomes of the invention substantially devoid of liquid in the interior thereof. By substantially devoid of liquid in the interior thereof, as used herein, it is meant liposomes having an interior that is at least 90% devoid of liquid, preferably at least 95% devoid of liquid, most preferably 100% devoid of liquid.

Unexpectedly, the drug containing liposomes prepared in accordance with the vacuum dried gas instillation method, and the drug containing gas filled liposomes substantially devoid of liquid in the interior thereof, possess a number of surprising yet highly beneficial characteristics. The liposomes of the invention exhibit intense echogenicity on ultrasound, will rupture on application of peak resonant frequency ultrasound (as well as other resonant freguencies of sufficient intensity and duration), are highly stable to pressure, and/or generally possess a long storage life, either when stored dry or suspended in a liquid medium.

The ecogenicity of the liposomes and the ability to rupture the liposomes at the peak resonant frequency using ultrasound permits the controlled delivery of drugs to a region of a patient by allowing the monitoring of the liposomes following administration to a patient to determine the presence of liposomes in a desired region, and the rupturing of the liposomes using ultrasound to release the drugs in the region. Preferably, the liposomes of the invention possess a reflectivity of greater than 2 dB, preferably between 4 dB and 20 dB. Within these ranges, the highest reflectivity for the liposomes of the invention is exhibited by the larger liposomes, by higher concentrations of liposomes, and/or when higher ultrasound frequencies are employed. Preferably, the liposomes of the invention have a peak resonant frequency of between 0.5 mHz and 10 mHz. Of course, the peak resonant frequency of the gas filled liposomes of the invention will vary depending on the diameter and, to some extent, the elasticity of the liposomes, with the larger and more elastic liposomes having a lower resonant frequency than the smaller and more elastic liposomes.

The stability of the liposomes of the invention is also of great practical importance. The subject liposomes tend to have greater stability during storage than other gas filled liposomes produced via known procedures such as pressurization or other techniques. At 72 hours after formation, for example, conventionally prepared gas containing liposomes often are essentially devoid of gas, the gas having diffused out of the liposomes and/or the liposomes having ruptured and/or fused, resulting in a concomitant loss in reflectivity. In comparison, drug containing gas filled liposomes of the present invention generally have a shelf life stability of greater than three weeks, preferably a shelf life stability of greater than four weeks, more preferably a shelf life stability of greater than five weeks, even more preferably a shelf life stability of greater than three months, and often a shelf life stability that is even much longer, such as over six months, twelve months, or even two years.

Also unexpected is the ability of the liposomes during the vacuum drying gas instillation process to fill with gas and resume their original circular shape, rather than collapse into a cup-shaped structure, as the prior art would cause one to expect. *See, e.g*., Crowe et al., Archives of Biochemistry and Biophysics, Vol. 242, pp. 240-247 (1985); Crowe et al., Archives of Biochemistry and Biophysics, Vol. 220, pp. 477-484 (1983); Fukuda et al., J. Am. Chem. Soc., Vol. 108, pp. 2321-2327 (1986); Regen et al., J. Am. Chem. Soc., Vol. 102, pp. 6638-6640 (1980).

The drug containing liposomes subjected to the vacuum drying gas instillation method of the invention may be prepared using any one of a variety of conventional liposome preparatory techniques which will be apparent to those skilled in the art. These techniques include freeze-thaw, as well as techniques such as sonication, chelate dialysis, homogenization, solvent infusion, microemulsification, spontaneous formation, solvent vaporization, French pressure cell technique, controlled detergent dialysis, and others, each involving preparing the liposomes in various fashions in a solution containing the desired drug so that the drug is encapsulated in the resultant liposome. Alternatively, drugs may be loaded into the liposomes using pH gradient techniques which, as those skilled in the art will recognize, is particularly applicable to drugs which either protonate or deprotonate at a particular pH. *See, e.g.*, Madden et al., Chemistry and Physics of Lipids, Vol. 53, pp. 37-46 (1990). The size of the drug containing liposomes can be adjusted, if desired, prior to vacuum drying and gas instillation, by a variety of procedures including extrusion, filtration, sonication, homogenization, employing a laminar stream of a core of liquid introduced into an immiscible sheath of liquid, and similar methods, in order to modulate resultant liposomal biodistribution and clearance. Extrusion under pressure through pores of defined size is, however, the preferred means of adjusting the size of the liposomes. The foregoing techniques, as well as others, are discussed, for example, in U.S. Patent No. 4,728,578; U.K. Patent Application GB 2193095 A; U.S. Patent No. 4,728,575; U.S. Patent No. 4,737,323; International Application PCT/US85/01161; Mayer et al., Biochimica et Biophysica Acta, Vol. 858, pp. 161-168 (1986); Hope et al., Biochimica et Biophysica Acta, Vol. 812, pp. 55-65 (1985); U.S. Patent No. 4,533,254; Mayhew et al., Methods in Enzymology, Vol. 149, pp. 64-77 (1987); Mayhew et al., Biochimica et Biophysica Acta, Vol 755, pp. 169-74 (1984); Cheng et al, Investigative Radiology, Vol. 22, pp. 47-55 (1987); PCT/US89/05040, U.S. Patent No. 4,162,282; U.S. Patent No. 4,310,505; U.S. Patent No. 4,921,706; and Linosome Technology, Gregoriadis, G., ed., Vol. I, pp. 29-31, 51-67 and 79-108 (CRC Press Inc., Boca Raton, FL 1984). Although any of a number of varying techniques can be employed, preferably the drug containing liposomes are prepared via microemulsification techniques. The liposomes produced by the various conventional procedures can then be employed in the vacuum drying gas instillation method of the present invention, to produce the drug containing liposomes of the present invention.

The materials which may be utilized in preparing liposomes to be employed in the vacuum drying gas instillation method of the present invention include any of the materials or combinations thereof known to those skilled in the art as suitable for liposome construction. The lipids used may be of either natural or synthetic origin. Such materials include, but are not limited to, lipids such as fatty acids, lysolipids, dipalmitoylphosphatidylcholine, phosphatidylcholine, phosphatidic acid, sphingomyelin, cholesterol, cholesterol hemisuccinate, tocopherol hemisuccinate, phosphatidylethanolamine, phosphatidyl-inositol, lysolipids, sphingomyelin, glycosphingolipids, glucolipids, glycolipids, sulphatides, lipids with ether and ester-linked fatty acids, polymerized lipids, diacetyl phosphate, stearylamine, distearoylphosphatidylcholine, phosphatidylserine, sphingomyelin, cardiolipin, phospholipids with short chain fatty acids of 6-8 carbons in length, synthetic phospholipids with asymmetric acyl chains (e.g., with one acyl chain of 6 carbons and another acyl chain of 12 carbons), 6-(5-cholesten-3β-yloxy)-1-thio-β-D-galactopyranoside, digalactosyldiglyceride, 6-(5-cholesten-3β-yloxy)hexyl-6-amino-6-deoxy-1-thio-β-D-galactopyranoside, 6-(5-cholesten-3β-yloxy)hexyl-6-amino-6-deoxyl-1-thio-α-D-mannopyranoside, dibehenoylphosphatidylcholine, dimyristoylphosphatidylcholine, dilauroylphosphatidylcholine, and dioleoylphosphatidylcholine, and/or combinations thereof. Other useful lipids or combinations thereof apparent to those skilled in the art which are in keeping with the spirit of the present invention are also encompassed by the present invention. For example, carbohydrates bearing lipids may be employed for *in vivo* targeting, as described in U.S. Patent No. 4,310,505. Of particular interest for use in the present invention are lipids which are in the gel state (as compared with the liquid crystalline state) at the temperature at which the vacuum drying gas instillation is performed. The phase transition temperatures of various lipids will be readily apparent to those skilled in the art and are described, for example, in Liposome Technology, Gregoriadis, G., ed., Vol. I, pp. 1-18 (CRC Press, Inc. Boca Raton, FL 1984). In addition, it has been found that the incorporation of at least a small amount of negatively charged lipid into any liposome membrane, although not required, is beneficial to providing highly stable liposomes. By at least a small amount, it is meant about 1 mole percent of the total lipid. Suitable negatively charged lipids will be readily apparent to those skilled in the art, and include, for example phosphatidylserine and fatty acids. Most preferred for the combined reasons of ultimate ability to rupture on application of resonant frequency ultrasound, ecogenicity and stability following the vacuum drying gas instillation process are liposomes prepared from dipalmitoylphosphatidylcholine.

Any of a variety of drugs may be encapsulated in the liposomes. By drugs, as used herein, it is meant any agent having beneficial and/or therapeutic effect on the patient. Suitable drugs include, but are not limited to: antineoplastic agents, such as platinum compounds (e.g., spiroplatin, cisplatin, and carboplatin), methotrexate, adriamycin, mitomycin, ansamitocin, blemoycin, cytosine arabinosine, arabinosyl, anenine, mercaptopolylysine, vincristine, busulfan, chlorambucil, melphasan (e.g., PAM, L-PAM or phenylalanine mustard), mercaptopurine, mitotane, procarbazine hydrochloride dacinomycin (actinomycin D), daunorubicin hydrochloride, doxorubicin hydrochloride, mitomycin, plicamycin (mithramycin), aminoglutethimide, estramustine phosphate sodium, flutamide, leuprolide acetate, megestrol acetate, tamoxifen citrate, testolactone, trilostane, amsacrine (m-AMSA), asparaginase (L-asparaginase) *Erwina* asparaginase, etoposide (VP-16), interferon α-2a, interferon α-2b, teniposide (VM-26), vinblastine sulfate (VLB), vincristine sulfate, bleomycin, bleomycin sulfate, methotrexate, adriamycin, cytosine arabinosine, and arabinosyl; biological response modifiers such as muramyldipeptide, muramyltripeptide, microbial cell wall components, lymphokines (e.g., bacterial endotoxin such as lipopolysaccharide, macrophage activation factor), sub-units of bacteria (such as Mycobacteria, Corynebacteria), the synthetic dipeptide N-acetyl-muramyl-L-alanyl-D-isoglutamine; genetic material such as nucleic acids, RNA, and DNA, of either natural or synthetic origin, including recombinant RNA and DNA; antifungal agents such as ketoconazole, nystatin, griseofulvin, flucytosone (5-fc), miconazole, amphotericin-β, ricin, and β-lactum antibiotics (e.g., sulfazecin); hormones such as growth hormone, melanocyte stimulating hormone, estradiol, beclomethasone dipropionate, betamethasone, betamethasone acetate and betamethasone sodium phosphate, vetamethasone disodium phosphate, vetamethasone sodium phosphate, cortisone acetate, dexamethasone, dexamethasone acetate, dexamethasone sodium phosphate, fluinsolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide and fludrocortisone acetate; vitamins such as cyanocobalamin and α-tocopherol; peptides, such as manganese super oxide dimutase; enzymes such as alkaline phosphatase; anti-allergic agents such as amelexanox; anti-coagulation agents such as phenprocoumon and heparin; circulatory drugs such as propranolol; metabolic potentiators such as glutathione; antituberculars such as para-aminosalicylic acid, isoniazid capreomycin sulfate cycloserine, ethambutol hydrochloride ethionamide, pyrazinamide, rifampin, and streptomycin sulfate; antivirals such as acyclovir, amantadine azidothymidine (AZT or Zidovudine), ribavirin and vidarabine monohydrate (adenine arabinoside, ara-A); antianginals such as diltiazem, nifedipine, verapamil, erythrityl tetranitrate, isosorbide dinitrate, nitroglycerin (glyceryl trinitrate) and pentaerythritol tetranitrate; anticoagulants such as phenprocoumon, heparin; antibiotics such as dapsone, chloramphenicol, neomycin, cefaclor, cefadroxil, cephalexin, cephradine erythromycin, clindamycin lincomycin, amoxicillin, ampicillin, bacampicillin, carbenicillin, cloxacillin, cyclacillin, picloxacillin, hetacillin, methicillin, nafcillin, oxacillin, penicillin G, penicillin V, ticarcillin fifampin and tetracycline; antiinflammatories such as difunisal, ibuprofen, indomethacin, meclofenamate, mefenamic acid, naproxen, oxyphenbutazone, phenylbutazone, piroxicam, culindac, tolmetin, aspirin and salicylates; antiprotozoans such as chloroquine, hydroxycloroquine, metronidazole, quinine and meglemine antimonate; antirheumatics such as penicillamine; narcotics such as paregoric; opiates such as codeine, heroin, methadone, morphine and opium; cardiac glycosides such as deslanoside, digitoxin, digoxin, digitalin and digitalis; neuromuscular blockers such as atracurium besylate, gallamine triethiodide, hexafluorenium bromide, metocurine iodide, pancuronium bromide, succinylcholine chloride (suxamethonium chloride), tubocurarine chloride and vecuronium bromide; sedatives (hypnotics) such as amobarbital, amobarbital sodium, aprobarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, phenobarbital sodium, secobarbital sodium, talbutal, temazepam and triazolam; local anesthetics such as bupivacaine hydrochloride, chloroprocaine hydrochloride, etidocaine hydrochloride, lidocaine hydrochloride, mepivacaine hydrochloride, procaine hydrochloride and tetracaine hydrochloride; general anesthetics such as droperidol, etomidate, fentanyl citrate with droperidol, ketamine hydrochloride, methohexital sodium and thiopental sodium; and radioactive particles or ions such as strontium, iodide rhenium and yttrium.

Similarly, prodrugs may be encapsulated in the liposomes, and are included within the ambit of the terms drug or drugs, as used herein. Prodrugs are well known in the art, and include, inactive drug precursors which, when exposed to high temperature, cavitation and/or pressure, in the presence of oxygen or otherwise, or when released from the liposomes, will form active drugs. Such prodrugs can be activated in the method of the invention, upon the application of ultrasound to the prodrug-containing liposomes with the resultant cavitation, heating, pressure, and/or release from the liposomes. Suitable prodrugs will be apparent to those skilled in the art, and are described, for example, in Sinkula et al., J. Pharm. Sci., Vol. 64, pp. 181-210 (1975). Prodrugs, for example, may comprise inactive forms of the active drugs wherein a chemical group is present on the prodrug which renders it inactive and/or confers solubility or some other property to the drug. In this form, the prodrugs are generally inactive, but once the chemical group has been cleaved from the prodrug, by heat, cavitation, pressure, and/or by enzymes in the surrounding environment or otherwise, the active drug is generated. Such prodrugs are well described in the art, and comprise a wide variety of drugs bound to chemical groups through bonds such as esters to short, medium or long chain aliphatic carbonates, hemiesters of organic phosphate, pyrophosphate, sulfate, amides, amino acids, azo bonds, carbamate, phosphamide, glucosiduronate, N-acetylglucosaminide and β-glucoside. Examples of drugs with the parent molecule and the reversible modification or linkage are as follows: convallatoxin with ketals, hydantoin with alkyl esters, chlorphenesin with glycine or alanine esters, acetaminophen with caffeine complex, acetylsalicylic acid with THAM salt, acetylsalicylic acid with acetamidophenyl ester, naloxone with sulfate ester, 15-methylprostaglandin F_{2α} with methyl ester, procaine with polyethylene glycol, erythromycin with alkyl esters, clindamycin with alkyl esters or phosphate esters, tetracycline with betaine salts, 7-acylaminocephalosporins with ring-substituted acyloxybenzyl esters, nandrolone with phenylproprionate decanoate esters, estradiol with enol ether acetal, methylprednisolone with acetate esters, testosterone with n-acetylglucosaminide glucosiduronate (trimethylsilyl) ether, cortisol or prednisolone or dexamethasone with 21-phosphate esters. Prodrugs may also be designed as reversible drug derivatives and utilized as modifiers to enhance drug transport to site-specific tissues. Examples of parent molecules with reversible modifications or linkages to influence transport to a site specific tissue and for enhanced therapeutic effect include isocyanate with haloalkyl nitrosurea, testosterone with propionate ester, methotrexate (3-5'-dichloromethotrexate) with dialkyl esters, cytosine arabinoside with 5'-acylate, nitrogen mustard (2,2'-dichloro-N-methyldiethylamine), nitrogen mustard with aminomethyl tetracycline, nitrogen mustard with cholesterol or estradiol or dehydroepiandrosterone esters and nitrogen mustard with azobenzene. As one skilled in the art would recognize, a particular chemical group to modify a given drug may be selected to influence the partitioning of the drug into either the membrane or the internal space of the liposomes. The bond selected to link the chemical group to the drug may be selected to have the desired rate of metabolism, e.g., hydrolysis in the case of ester bonds in the presence of serum esterases after release from the gas filled liposomes. Additionally, the particular chemical group may be selected to influence the biodistribution of the drug employed in the gas filled drug carrying liposome invention, e.g., N,N-bis(2-chloroethyl)-phosphorodiamidic acid with cyclic phosphoramide for ovarian adenocarcinoma. Additionally, the prodrugs employed within the gas filled liposomes may be designed to contain reversible derivatives which are utilized as modifiers of duration of activity to provide, prolong or depot action effects. For example, nicotinic acid may be modified with dextran and carboxymethlydextran esters, streptomycin with alginic acid salt, dihydrostreptomycin with pamoate salt, cytarabine (ara-C) with 5'-adamantoate ester, ara-adenosine (ara-A) with 5-palmitate and 5'-benzoate esters, ampho- tericin-β with methyl esters, testosterone with 17-β-alkyl esters, estradiol with formate ester, prostaglandin with 2-(4-imidazolyl)ethylamine salt, dopamine with amino acid amides, chloramphenicol with mono- and bis(trimethylsilyl) ethers, and cycloguanil with pamoate salt. In this form, a depot or reservoir of long-acting drug may be released *in vivo* from the gas filled prodrug bearing liposomes. In addition, compounds which are generally thermally labile may be utilized to create toxic fee radical compounds. Compounds with azolinkages, peroxides and disulfide linkages which decompose with high temperature are preferred. With this form of prodrug, azo, peroxide or disulfide bond containing compounds are activated by cavitation and/or increased heating caused by the interaction of high energy sound with the gas filled liposomes to create cascades of free radicals from these prodrugs entrapped therein. A wide variety of drugs or chemicals may constitute these prodrugs, such as azo compounds, the general structure of such compounds being R-N=N-R, wherein R is a hydrocarbon chain, where the double bond between the two nitrogen atoms may react to create free radical products in *vivo.* Exemplary drugs or compounds which may be used to create free radical products include azo containing compounds such as azobenzene, 2,2'-azobisisobutyronitrile, azodicarbonamide, azolitmin, azomycin, azosemide, azosulfamide, azoxybenzene, aztreonam, sudan III, sulfachrysoidine, sulfamidocnrysoidine and sulfasalazine, compounds containing disulfide bonds such as sulbentine, thiamine disulfide, thiolutin, thiram, compounds containing peroxides such as hydrogen peroxide and benzoylperoxide, 2,2'-azobis(2-amidopropane) dihydrochloride, and 2,2'-azobis(2,4-dimethylvaleronitrile). A gas filled liposome filled with oxygen gas should create extensive free radicals with cavitation. Also, metal ions from the transition series, especially manganese, iron and copper can increase the rate of formation of reactive oxygen intermediates from oxygen. By encapsulating metal ions within the liposomes, the formation of free radicals in *vivo* can be increased. These metal ions may be incorporated into the liposomes as free salts, as complexes, e.g., with EDTA, DTPA, DOTA or desferrioxamine, or as oxides of the metal ions. Additionally, derivatized complexes of the metal ions may be bound to the lipid head groups, or lipophilic complexes of the ions may be incorporated into the lipid bilayer. When exposed to thermal stimulation, e.g., cavitation, these metal ions then will increase the rate of formation of reactive oxygen intermediates. Further, radiosensitizers such as metronidazole and misonidazole may be incorporated into the gas filled liposomes to create free radicals on thermal stimulation.

By way of an example of the use of prodrugs, an acylated chemical group may be bound to a drug via an ester linkage which would readily cleave *in vivo* by enzymatic action in serum. The acylated prodrug is incorporated into the gas filled liposome of the invention. The liposomes may also be designed so that there is a symmetric or an asymmetric distribution of the drug both inside and outside of the liposome. When the gas filled liposome is popped by the sonic pulse from the ultrasound, the prodrug encapsulated by the liposome will then be exposed to the serum. The ester linkage is then cleaved by esterases in the serum, thereby generating the drug.

Similarly, ultrasound may be utilized not only to pop the gas filled liposome, but also to cause thermal effects which may increase the rate of the chemical cleavage and the release of the active from the prodrug.

As one skilled in the art will recognize, the particular chemical structure of the drugs may be selected or modified to achieve desired solubility such that the drug may either be encapsulated within the internal aqueous space of the liposome or into the lipid membrane. The membrane bound drug may bear one or more acyl chains such that, when the bubble is popped or heated or via cavitation, the acylated drug may then leave the membrane and/or the drug may be cleaved from the acyl chains chemical group. Similarly, other drugs may be formulated with a hydrophobic group which is aromatic or sterol in structure to incorporate into the membrane.

To prepare the drug containing liposomes, and by way of general guidance, dipalmitoylphosphatidylcholine liposomes, for example,may be prepared by suspending dipalmitoyl- phosphatidylcholine lipids in phosphate buffered saline or water containing the drug to be encapsulated, and heating the lipids to about 50°C, a temperature which is slightly above the 45°C temperature required for transition of the dipalmitoylphosphatidylcholine lipids from a gel state to a liquid crystalline state, to form drug containing liposomes. To prepare multilamellar vesicles of a rather heterogeneous size distribution of around 2 microns, the liposomes may then be mixed gently by hand while keeping the liposome solution at a temperature of about 50°C. The temperature is then lowered to room temperature, and the liposomes remain intact. Extrusion of dipalmitoylphosphatidylcholine liposomes through polycarbonate filters of defined size may, if desired, be employed to make liposomes of a more homogeneous size distribution. A device useful for this technique is an extruder device (Extruder Device™, Lipex Biomembranes, Vancouver, Canada) equipped with a thermal barrel so that extrusion may be conveniently accomplished above the gel state-liquid crystalline transition temperature for lipids.

For lipophilic drugs which are sparingly soluble in aqueous media, such drugs may be mixed with the lipids themselves prior to forming the liposomes. For example, amphotericin may be suspended with the dried lipids (e.g., 8:2 molar ratio of egg phosphatidylcholine and cholesterol in chloroform and mixed with the lipids). The chloroform is then evaporated (note that other suitable organic solvents may also be used, such as ethanol or ether) and the dried lipids containing a mixture of the lipophilic drug are then resuspended in aqueous media, e.g., sterile water or physiologic saline. This process may be used for a variety of lipophilic drugs such as corticosteroids to incorporate lipophilic drugs into the liposome membranes. The resulting liposomes are then dried, subjected to the vacuum gas instillation method as described above.

Alternatively, and again by way of general guidance, conventional freeze-thaw procedures may be used to produce either oligolamellar or unilamellar dipalmitoylphosphatidyl- choline liposomes. After the freeze-thaw procedures, extrusion procedures as described above may then be performed on the liposomes.

The drug containing liposomes thus prepared may then be subjected to the vacuum drying gas instillation process of the present invention, to produce the drug containing vacuum dried gas instilled liposomes, and the drug containing gas filled liposomes at least 90% devoid of liquid in the interior thereof, of the invention. In accordance with the process of the invention, the drug containing liposomes are placed into a vessel suitable for subjecting to the liposomes to negative pressure (that is, reduced pressure or vacuum conditions). Negative pressure is then applied for a time sufficient to remove substantially all liquid from the liposomes, thereby resulting in substantially dried liposomes. As those skilled in the art would recognize, once armed with the present disclosure, various negative pressures can be employed, the important parameter being that substantially all of the liquid has been removed from the liposomes. Generally, a negative pressure of at least 700 mm Hg and preferably in the range of between 700 mm Hg and 760 mm Hg (gauge pressure) applied for 24 to 72 hours, is sufficient to reaove at least 90% of the liquid from the liposomes. Other suitable pressures and time periods will be apparent to those skilled in the art, in view of the disclosures herein.

Finally, a selected gas is applied to the liposomes to instill the liposomes with gas until ambient pressures are achieved, thereby resulting in the drug containing vacuum dried gas instilled liposomes of the invention, and in the drug containing gas filled liposomes substantially devoid of liquid in the interior thereof. Preferably, gas instillation occurs slowly, that is, over a time period of at least 4 hours, most preferably over a time period of between 4 and 8 hours. Various biocompatible gases may be employed. Such gases include air, nitrogen, carbon dioxide, oxygen, argon, xenon, neon, helium, or any and all combinations thereof. Other suitable gases will be apparent to those skilled in the art, the gas chosen being only limited by the proposed application of the liposomes.

The above described method for production of liposomes is referred to hereinafter as the vacuum drying gas instillation process.

If desired, the liposomes may be cooled, prior to subjecting the liposomes to negative pressure, and such cooling is preferred. Preferably, the liposomes are cooled to below 0°C, more preferably to between -10°C and -20°C, and most preferably to -10°C, prior to subjecting the liposomes to negative pressure. Upon reaching the desired negative pressure, the liposomes temperature is then preferably increased to above 0°C, more preferably to between 10°C and 20°C, and most preferably to 10°C, until substantially all of the liquid has been removed from the liposomes and the negative pressure is discontinued, at which time the temperature is then permitted to return to room temperature.

If the liposomes are cooled to a temperature below 0°C, it is preferable that the vacuum drying gas instillation process be carried out with liposomes either initially prepared in the presence of cryoprotectants, or liposomes to which cryoprotectants have been added prior to carrying out the vacuum drying gas instillation process of the invention. Such cryoprotectants, while not mandatorily added, assist in maintaining the integrity of liposome membranes at low temperatures, and also add to the ultimate stability of the membranes. Preferred cryoprotectants are trehalose, glycerol, polyethyleneglycol (especially polyethyleneglycol of molecular weight 400), raffinose, sucrose and sorbitol, with trehalose being particularly preferred.

It has also been surprisingly discovered that the liposomes of the invention are highly stable to changes in pressure. Because of this characteristic, extrusion of the liposomes through filters of defined pore size following vacuum drying and gas instillation can be carried out, if desired, to create liposomes of relatively homogeneous and defined pore size.

For storage prior to use, the drug containing liposomes of the present invention may be suspended in an aqueous solution, such as a saline solution (for example, a phosphate buffered saline solution), or simply water, and stored preferably at a temperature of between 2°C and 10°C, preferably at 4°C. Preferably, the water is sterile. Most preferably, the liposomes are stored in a hypertonic saline solution (e.g., 0.3 to 0.5% NaCl), although, if desired, the saline solution may be isotonic. The solution also may be buffered, if desired, to provide a pH range of pH 6.8 to pH 7.4. Suitable buffers include, but are not limited to, acetate, citrate, phosphate and bicarbonate. Dextrose may also be included in the suspending media. Preferably, the aqueous solution is degassed (that is, degassed under vacuum pressure) prior to suspending the liposomes therein. Bacteriostatic agents may also be included with the liposomes to prevent bacterial degradation on storage. Suitable bacteriostatic agents include but are not limited to benzalkonium chloride, benzethonium chloride, benzoic acid, benzyl alcohol, butylparaben, cetylpyridinium chloride, chlorobutanol, chlorocresol, methylparaben, phenol, potassium benzoate, potassium sorbate, sodium benzoate and sorbic acid. One or more antioxidants may further be included with the gas filled liposomes to prevent oxidation of the lipid. Suitable antioxidants include tocopherol, ascorbic acid and ascorbyl palmitate. Liposomes prepared in the various foregoing manners may be stored for at least several weeks or months. Liposomes of the present invention may alternatively, if desired, be stored in their dried, unsuspended form, and such liposomes also have a shelf life of greater than several weeks or months. Specifically, the liposomes of the present invention, stored either way, generally have a shelf life stability of greater than three weeks, preferably a shelf life stability of greater than four weeks, more preferably a shelf life stability of greater than five weeks, even more preferably a shelf life stability of greater than three months, and often a shelf life stability that is even much longer, such as over six months, twelve months or even two years.

There is shown in Figure 1 a preferred apparatus for vacuum drying liposomes and instilling a gas into the dried liposomes. The apparatus is comprised of a vessel 8 for containing drug containing liposomes 19. If desired, the apparatus may include an ice bath 5 containing dry'ice 17 surrounding the vessel 8. The ice bath 5 and dry ice 17 allow the liposomes to be cooled to below 0°C. A vacuum pump 1 is connected to the vessel 8 via a conduit 15 far applying a sustained negative pressure to the vessel. In the preferred embodiment, the pump 1 is capable of applying a negative pressure of at least 700 mm Hg, and preferably a negative pressure in the range of 700 mm Hg to 760 mm Hg (gauge pressure). A manometer 6 is connected to the conduit 15 to allow monitoring of the negative pressure applied to the vessel 8.

In order to prevent liquid removed from the liposomes from entering the pump 1, a series of traps are connected to the conduit 15 to assist in collecting the liquid (and liquid vapor, all collectively referred to herein as liquid) drawn from the liposomes. In a preferred embodiment, two traps are utilized. The first trap is preferably comprised of a flask 7 disposed in an ice bath 4 with dry ice 17. The second trap is preferably comprised of a column 3 around which tubing 16 is helically arranged. The column 3 is connected to the conduit 15 at its top end and to one end of the tubing 16 at its bottom end. The other end of the tubing 16 is connected to the conduit 15. As shown in Figure 1, an ice bath 2 with dry ice 17 surrounds the column 3 and tubing 16. If desired, dry ice 17 can be replaced with liquid nitrogen, liquid air or other cryogenic material. The ice baths 2 and 4 assist in collecting any liquid and condensing any liquid vapor drawn from the liposomes for collection in the traps. In preferred embodiments of the present invention the ice traps 2 and 4 are each maintained at a temperature of least -70°C.

A stopcock 14 is disposed in the conduit 15 upstream of the vessel 8 to allow a selected gas to be introduced into the vessel 8 and into the liposomes 19 from gas bottle 18.

Apparatus are utilized by placing the'drug containing liposomes 19 into vessel 8. In a preferable embodiment, ice bath 5 with dry ice 17 is used to lower the temperature of the liposomes to below 0°C, more preferably to between -10°C and -20°C, and most preferably to -10°C With stopcocks 14 and 9 closed, vacuum pump 1 is turned on. Stopcocks 10, 11, 12 and 13 are then carefully opened to create a vacuum in vessel 8 by means of vacuum pump 1. The pressure is gauged by means of manometer 6 until negative pressure of at least 700 mm Hg, and preferably in the range of between 700 mm Hg and 760 mm Hg (gauge pressure) is achieved. In preferred embodiments of the present invention vessel 7, cooled by ice bath 4 with dry ice 17, and column 3 and coil 16, cooled by ice bath 2 with dry ice 17, together or individually condense liquid vapor and trap liquid drawn from the liposomes so as to prevent such liquids and liquid vapor from entering the vacuum pump 1. In preferred embodiments of the present invention, the temperature of ice traps 2 and 4 are each maintained at a temperature of at least -70°C. The desired negative pressure is generally maintained for at least 24 hours as liquid and liquid vapor is removed from the liposomes 19 in vessel 8 and frozen in vessels 3 and 7. Pressure within the system is monitored using manometer 6 and is generally maintained for 24 to 72 hours, at which time at least 90% of the liquid has been removed from the liposomes. At this point, stopcock 10 is slowly closed and vacuum pump 1 is turned off. Stopcock 14 is then opened gradually and gas is slowly introduced into the system from gas bottle 18 through stopcock 14 via conduit 15 to instill gas into the drug containing liposomes 19 in vessel 8. Preferably the gas instillation occurs slowly over a time period of at least 4 hours, most preferably over a time period of between 4 and 8 hours, until the system reaches ambient pressure.

The drug containing vacuum dried gas instilled liposomes and the drug containing gas filled liposomes at least 90% devoid of liquid in the interior thereof, of the present invention, have superior characteristics as drug delivery vehicles. Specifically, the present invention may be employed in the controlled delivery of drugs to a region of a patient wherein the patient is administered the drug containing liposome of the present invention, the liposomes are monitored using ultrasound to determine the presence of the liposomes in the region, and the liposomes are then ruptured using ultrasound to release the drugs in the region. The patient may be any type of mammal, but is most preferably human. By region of a patient, it is meant the whole patient, or a particular area or portion of the patient. For example, by using the method of the invention, drug delivery may be effected in a patient's heart, and a patient's vasculature (that is, venous or arterial systems). The invention is also particularly useful in delivering drugs to a patient's left heart, a region not easily reached heretofore with drug delivery. Drugs may also be easily delivered to the liver, spleen and kidney regions of a patient, as well as other regions, using the present methods.

The rupturing of the drug containing liposomes of the invention is surprisingly easily carried out by applying ultrasound of a certain frequency to the region of the patient where therapy is desired, after the liposomes have been administered to or have otherwise reached that region. Specifically, it has been unexpectedly found that when ultrasound is applied at a frequency corresponding to the peak resonant frequency of the drug containing gas filled liposomes, the liposomes will rupture and release their contents. The peak resonant frequency can be determined either *in vivo* or *in vitro,* but preferably *in vivo*, by exposing the liposomes to ultrasound, receiving the reflected resonant frequency signals and analyzing the spectrum of signals received to determine the peak, using conventional means. The peak, as so determined, corresponds to the peak resonant frequency (or second harmonic, as it is sometimes termed). The gas filled liposomes will also rupture when exposed to non-peak resonant frequency ultrasound, however, the intensity (wattage) and duration (time) must be higher in order to cause the liposomes to rupture. This higher energy results in greatly increased heating, which may not be desireable. By adjusting the frequency of the energy to match the peak resonant frequency, the efficiency of rupture and drug release is improved, appreciable tissue heating does not generally occur, (frequently no increase in temperature above about 2°C), and less overall energy is required. Thus, application of ultrasound at the peak resonant frequency while not required is most preferred.

Any of the various types of diagnostic ultrasound imaging devices may be employed in the practive of the invention, the particular type or model of the device not being critical to the method of the invention. Also suitable are devices designed for administering ultrasonic hyperthermia, such devices being described in U.S. Patent Nos. 4,620,546, 4,658,828, and 4,586,512. Preferably, the device employs a resonant frequency (RF) spectral analyzer. Ultrasound is generally initiated at lower intensity and duration, preferably at peak resonant frequency, and then intensity, time, and/or resonant frequency increased until liposomal rupturing occurs.

Although application of the various principles will be readily apparent to one skilled in the art, once armed with the present disclosure, by way of general guidance, for gas filled liposomes of 1.5 to 2.0 microns diameter, the resonant frequency will generally be in the range of about 7.5 megahertz. By adjusting the focal zone to the center of the target tissue (e.g., the tumor) the gas filled liposomes can be visualized under real time ultrasound as they accumulate within the target tissue. Using the 7.5 megahertz curved array transducer as an example, adjusting the power delivered to the transducer to maximum and adjusting the focal zone within the target tissue, the spatial peak temporal average (SPTA) power will then be a maximum of approximately 5.31 mW/cm² in water. This power will cause some release of drug from the gas filled liposomes, but much greater release can be accomplished by using higher power. By switching the transducer to the doppler mode, higher power outputs are available, up to 2.5 watts per cm² from the same transducer. With the machine operating in doppler mode, the power can be delivered to a selected focal zone within the target tissue and the gas filled liposomes can be made to release their drugs. Selecting the transducer to match the resonant frequency of the gas filled liposomes will make this process of drug release even more efficient. For larger diameter gas filled liposomes, e.g., greater than 3 microns in size, a lower frequency transducer may be more effective in accomplishing drug release. For example, a lower frequency transducer of 3.5 megahertz (20 mm curved array model) may be selected to correspond to the resonant frequency of the gas filled liposomes. Using this transducer, 101.6 milliwatts per cm² may be delivered to the focal spot, and switching to doppler mode will increase the power output (SPTA) to 1.02 watts per cm². To use the phenomenon of cavitation to release and/or activate the drugs/prodrugs within the gas filled liposomes lower frequency energies may be used, as cavitation occurs more effectively at lower frequencies. Using a 0.757 megahertz transducer driven with higher voltages (as high as 300 volts) cavitation of solutions of gas filled liposomes will occur at thresholds of about 5.2 atmospheres.

Liposomes of the present invention may be of varying sizes, but preferably are of a size range wherein they have a mean outside diameter between 30 nanometers and 10 microns, with the preferable mean outside diameter being about 2 microns. As is known to those skilled in the art, liposome size influences biodistribution and, therefore, different size liposomes may be selected for various purposes. For intravascular use, for example, liposome size is generally no larger than 5 microns, and generally no smaller than 30 nanometers, in mean outside diameter. To provide drug delivery to organs such as the liver and to allow differentiation of tumor from normal tissue, smaller liposomes, between 30 nanometers and 100 nanometers in mean outside diameter, are useful. With the smaller liposomes, resonant frequency ultrasound will generally be higher than for the larger liposomes.

The gas filled drug containing liposomes of the present invention may be used with any of the various types of ultrasound imaging devices designed for administering ultrasonic hyperthermia. Such devices are well known and are described in U.S. Patent Nos. 4,620,546, 4,658,828 and 4,586,512, and are commonly employed in physical therapy and sports medicine. It is preferable to have direct access to the resonant frequency data from the ultrasonic data using commercially available software, rather than to reformat this data (e.g., via Fourrier transform) into two dimensional or three dimensional images, to determine the peak resonant frequency. Also, the transducer probes may be external or may be implanted, if desired.

As one skilled in the art would recognize, administration of drug delivery systems of the present invention may be carried out in various fashions, such as intravascularly, intralymphatically, parenterally, subcutaneously, intramuscularly, intraperitoneally, interstitially, hyperbarically, orally, or intratumorly, using a variety of dosage forms. One preferred route of administration is intravascularly. For intravascular use, the drug delivery system is generally injected intravenously, but may be injected intraarterially as well. The useful dosage to be administered and the mode of administration will vary depending upon the age, weight, and mammal to be treated, and the particular therapeutic application intended. Typically, dosage. is initiated at lower levels and increased until the desired therapeutic effect is achieved. Generally, the drug delivery systems of the invention are administered in the form of an aqueous suspension such as in water or a saline solution (e.g., phosphate buffered saline). Preferably, the water is sterile. Also, preferably the saline solution is a hypertonic saline solution (e.g., 0.3 to 0.5% NaCl), although, if desired, the saline solution may be isotonic. The solution may also be buffered, if desired, to provide a pH range of pH 6.8 to pH 7.4. In addition, dextrose may be preferably included in the media. Preferably, the aqueous solution is degassed (that is, degassed under vacuum pressure) prior to suspending the liposomes therein.

The liposomes of the present invention are believed to differ from the liposomes of the prior art in a number of respects, both in physical and in functional. characteristics. For example, the liposomes of the invention are substantially devoid of liquid in the interior thereof. By definition, liposomes in the prior art have been characterized by the presence of an aqueous medium. *See, e.g.,* Dorland's Illustrated Medical Dictionary, p. 946, 27th ed. (W.B. Saunders Company, Philadelphia 1988). Moreover, the present liposomes surprisingly exhibit intense ecogenicity on ultrasound, are susceptible to rupture upon application of ultrasound at the peak resonant frequency of the liposomes, and possess a long storage life, characteristics of great benefit to the use of the liposomes as drug delivery systems.

There are various other applications for liposomes of the invention, beyond those described in detail herein. Such additional uses, for example, include such applications as hyperthermia potentiators for ultrasound and as contrast agents for ultrasonic imaging.

The present invention is further described in the following examples. Examples 1-10 are prophetic examples that describe the preparation, testing and use of the drug containing vacuum dried gas instilled liposomes, the gas filled liposomes being at least 90% devoid of any liquid in the interior thereof. The following examples should not be construed as limiting the scope of the appended claims.

### EXAMPLES

### Example 1

Dipalmitoylphosphatidylcholine (1 gram) is suspended in 10 ml phosphate buffered saline containing the drug adriamycin, the suspension is heated to about 50°C, and then is swirled by hand in a round bottom flask for about 30 minutes. The heat source is removed, and the suspension is swirled for two additional hours, while allowing the suspension to cool to room temperature, to form drug containing liposomes.

The liposomes thus prepared are placed in a vessel in an apparatus similar to that shown in Figure 1, cooled to about -10°C, and are then subjected to high negative vacuum pressure. The temperature of the liposomes is then raised to about 10°C. High negative vacuum pressure is maintained for about 48 hours. After about 48 hours, nitrogen gas is gradually instilled into the chamber over a period of about 4 hours after which time the pressure is returned to ambient pressure. The resulting drug containing vacuum dried gas instilled liposomes, the gas filled liposomes being substantially devoid of any liquid in the interior thereof, are then suspended in 10 cc of phosphate buffered saline, and then stored at about 4°C for about three months.

### Example 2

To test the liposomes of Example 1 ultrasonographically, a 250 mg sample of these liposomes is suspended in 300 cc of degassed phosphate buffered saline (that is, degassed under vacuum pressure). The liposomes are then scanned *in vitro* at varying time intervals with a 7.5 mHz transducer using an Acoustic Imaging Model 5200 scanner
(Acoustic Imaging, Phoenix, AZ) and employing the system test software to measure dB reflectivity. The system is standardized prior to testing the liposomes with a phantom of known acoustic impedance. Good dB reflectivity of the liposomes is shown.

### Example 3

Dipalmitoylphosphatidylcholine (1 gram) and the cryoprotectant trehalose (1 gram) are suspended in 10 ml phosphate buffered saline containing the drug amphotericin-B, the suspension is heated to about 50°C, and then is swirled by hand in a round bottom flask for about 30 minutes. The heat source is removed, and the suspension is swirled for about two additional hours, while allowing the suspension to cool to room temperature, to form liposomes. The liposomes thus prepared are then vacuum dried and gas instilled, substantially following the procedures shown in Example 1, resulting in drug containing vacuum dried gas instilled liposomes, the gas filled liposomes being substantially devoid of any liquid in the interior thereof. The liposomes are then suspended in 10 cc of phosphate buffered saline, and then stored at about 4°C for several weeks.

### Example 4

To test the liposomes of Example 3 ultrasonographically, the procedures of Example 2 are substantially followed. Good dB reflectivity of the liposomes is shown.

### Example 5

Dipalmitoylphosphatidylcholine (1 gram) is suspended in 10 ml phosphate buffered saline containing the drug cytosine arabinosine, the suspension is heated to about 50°C, and then swirled by hand in a round bottom flask for about 30 minutes. The suspension is then subjected to 5 cycles of extrusion through an extruder device jacketed with a thermal barrel (Extruder Device™, Lipex Biomembranes, Vancouver, Canada), both with and without conventional freeze-thaw treatment prior to extrusion, while maintaining the temperature at about 50°C. The heat source is removed, and the suspension is swirled for about two additional hours, while allowing the suspension to cool to room temperature, to form liposomes.

The liposomes thus prepared are then vacuum dried and gas instilled, substantially following the procedures shown in Example 1, resulting in drug containing vacuum dried gas instilled liposomes, the gas filled liposomes being substantially devoid of any liquid in the interior thereof. The liposomes are then suspended in 10 cc of phosphate buffered saline, and then stored at about 4°C for several weeks.

### Example 6

To test liposomes of Example 5 ultrasonographically, the procedures of Example 2 are substantially followed. Good dB reflectivity of the liposomes is shown.

### Example 7

In order to test the stability of the drug containing liposomes of the invention, the liposomes suspension of Example 1 is passed through 2 micron polycarbonate filters in an extruder device (Extruder Device™, Lipex Biomembranes, Vancouver, Canada) five times at a pressure of about 600 psi. After extrusion treatment, the liposomes are studied ultrasonographically, as described in Example 2. Surprisingly, even after extrusion under high pressure, the liposomes of the invention substantially retain their echogenicity.

### Example 8

The liposomes of Example 1 are scanned by ultrasound using transducer frequencies varying from 3 to 7.5 mHz. The results indicate that at a higher frequency of ultrasound, the echogenicity decays more rapidly, reflecting a relatively high resonant frequency and higher energy associated with the higher frequencies.

### Example 9

A patient with cancer is given an intravenous drug containing vacuum dried gas instilled liposomes, the gas filled liposomes being substantially devoid of any liquid in the interior thereof. The drug contained in the liposomes is adriamycin. As the intravenous injection is administered, the tumor is scanned ultrasonographically and via an automated software program, and the resonant frequency of the liposomes is determined. Ultrasonic energy is then focused into the tumor at the peak resonant frequency of the liposomes. The amount of ultrasonic energy is insufficient to cause any appreciable tissue heating (that is, no change in temperature greater than 2°C), however, this energy is sufficient to cause the liposomes to pop and release the adriamycin at the tumor site. In so doing, local drug delivery is accomplished using the liposomes with ultrasound.

### Example 10

In a patient with a severe localized fungal infection, drug containing vacuum dried gas instilled liposomes, the gas filled liposomes being substantially devoid of any liquid in the interior thereof, are injected intravenously and ultrasound is used in a fashion substantially similar to that described in Example 9 to accomplish local drug delivery. The drug amphotericin-B, which the liposomes contain, is effectively delivered to the site of the infection.

Various modifications of the invention within the scope of the appended claims in addition to those shown and described herein will be apparent to those skilled in the art from the foregoing description.

## Claims

1. A drug delivery system comprising gas filled liposomes at least 90% devoid of liquid in the interior thereof and having encapsulated therein a drug, said liposomes being prepared by a vacuum drying gas instillation method comprising the steps of:
(i) applying negative pressure to a vessel containing liposomes having encapsulated therein said drug;
(ii) incubating said liposomes under said negative pressure, at a temperature above 0°C, for a time sufficient to remove at least 90% of the liquid from said liposomes; and
(iii) instilling gas into said liposomes until ambient pressures are achieved, to produce gas-filled liposomes which contain said drug.

2. A drug delivery system comprising echogenic, gas-filled liposomes at least 90% devoid of liquid in the interior thereof having a gas and a drug encapsulated therein, wherein said liposomes are capable of being induced to rupture or release said drug upon the application of ultrasound.

3. A drug delivery system of claim 1 or claim 2 wherein said gas filled liposomes are comprised of lipid materials selected from the group consisting of fatty acids, lysolipids, dipalmitoylphosphatidylcholine, phosphatidylcholine, phosphatidic acid, sphingomyelin, cholesterol, cholesterol hemisuccinate, tocopherol hemisuccinate, phosphatidyl-ethenolamine, phosphatidylinositol, lysolipids, glycosphingolipids, glucolipids, glycolipids, sulphatides, lipids with ether and ester-linked fatty acids, and polymerized lipids.

4. A drug delivery system of claim 2 wherein said gas filled liposomes are comprised of dipalmitoylphosphatidylcholine.

5. A drug delivery system of any preceding claim wherein said gas filled liposomes are filled with a gas selected from the group consisting of air, nitrogen, carbon dioxide, oxygen, argon, xenon, helium, and neon.

6. A drug delivery system of any preceding claim wherein said gas filled liposomes are stored suspended in an aqueous medium.

7. A drug delivery system of claims 1 to 5, wherein said gas filled liposomes are stored dry.

8. A drug delivery system of any preceding claim wherein said gas filled liposomes have a stability of greater than three weeks.

9. A drug delivery system of any preceding claim wherein said gas filled liposomes have a reflectivity of greater than about 2 dB.

10. A drug delivery system of claim 9 wherein said gas filled liposomes have a reflectivity of between 2 dB and 20 dB.

11. A method for preparing a drug delivery system comprising the following steps:
(i) applying negative pressure to a vessel containing liposomes having encapsulated therein a drug;
(ii) incubating said liposomes under said negative pressure, at a temperature above 0°C, for a time sufficient to remove at least 90% of the liquid from said liposomes; and
(iii) instilling gas into said liposomes until ambient pressures are achieved, to produce gas-filled liposomes which contain said drug.

12. , A method of claim 11 further comprising allowing said liposomes to cool prior to and during step (i) to a temperature between -10°C and -20°C, allowing said liposomes to warm during step (ii) to a temperature between 10°C and 20°C, and allowing said liposomes to warm during step (iii) to ambient temperatures.

13. A method of claim 11 or 12 wherein said negative pressure is between 700 mm Hg and 760 mm Hg and is applied for 24 to 72 hours.

14. A method of claim 11, 12 or 13 wherein said gas is instilled into said liposomes over a period of 4 to 8 hours.

15. A method of any one of claims 11 to 14 further comprising, after step (iii), extruding said liposomes through at least one filter of a selected pore size.

16. A method for preparing drug delivery systems for use in conjunction with ultrasonic imaging comprising the following steps:
(i) allowing liposomes having encapsulated therein a drug to cool to a temperature between -10°C and -20°C;
(ii) placing said liposomes in a vessel under a negative pressure of between 93.3 kPa (700 mm Hg) to 101.3 kPa (760 mm Hg);
(iii) allowing said liposomes to warm to a temperature of between 10°C and 20°C;
(iv) incubating said liposomes under said negative pressure for 24 to 72 hours to remove at least 90% of the liquid from said liposomes; and
(v) instilling gas into said liposomes over a period of 4 to 8 hours until ambient pressures are achieved, while allowing said liposomes to warm to ambient temperature.

17. A method of any one of claims 11 to 16, wherein said gas is selected from the group consisting of air, nitrogen, carbon dioxide, oxygen, argon, xenon, neon, and helium.

18. A method of claim 16 further comprising, after step (iv), extruding said liposomes through at least one filter of a selected pore size.

19. Use of a drug delivery system according to any one of claims 1 to 10 in the preparation of a composition for use in delivering a drug to an internal bodily region of a patient, wherein the presence of the liposomes in the region is monitored using ultrasound and the drug is released from the liposomes in the said region by rupturing the liposomes using ultrasound.

20. Use according to claim 19, wherein the drug is delivered to the left heart of a patient.

## Patentansprüche

1. Arzneimittel-Freisetzungssystem, welches gasgefüllte Liposomen umfaßt, wobei mindestens 90% ohne Flüssigkeit in dem Inneren davon sind und darin eingekapselt ein Arzneimittel aufweisen, wobei die Liposomen durch ein Vakuumtrockengaseinbringverfahren hergestellt werden, welches die Schritte umfaßt
(i) das Anlegen von negativem Druck an ein Gefäß, welches Liposomen mit dem darin eingekapselten Arzneimittel enthält,
(ii) das Inkubieren der Liposomen unter dem negativen Druck bei einer Temperatur von oberhalb 0°C für eine Zeitdauer, die ausreichend ist, mindestens 90% der Flüssigkeit von den Liposomen zu entfernen, und
(iii) das Einbringen von Gas in die Liposomen, bis Umgebungsdrücke erreicht werden, wodurch gasgefüllte Liposomen, die das Arzneimittel enthalten, erzeugt werden.

2. Arzneimittel-Freisetzungssystem, welches echogene, gasgefüllte Liposomen umfasst, wobei mindestens 90% ohne Flüssigkeit in dem Inneren davon ein Gas und ein Arzneimittel darin eingekapselt aufweisen, wobei die Liposomen befähigt sind, veranlaßt zu werden, nach der Anwendung von Ultraschall zu brechen oder das Arzneimittel freizusetzen.

3. Arzneimittelfreisetzungssystem nach Anspruch 1 oder Anspruch 2, wobei die gasgefüllten Liposomen Lipidmaterialien, ausgewählt aus der Gruppe, bestehend aus Fettsäuren, Lysolipiden, Dipalmitoylphosphatidylcholin, Phosphatidylcholin, Phosphatidsäure, Sphingomyelin, Cholesterin, Cholesterinhemisuccinat, Tocopherolhemisuccinat, Phosphatidyl-Ethanolamin, Phosphatidylinositol, Lysolipiden, Glycosphingolipiden, Glucolipiden, Glycolipiden, Sulfatiden, Lipiden mit Ether- und Ester-gebundenen Fettsäuren und polymerisierten Lipiden, umfassen.

4. Arzneimittel-Freisetzungssystem nach Anspruch 2, wobei die gasgefüllten Liposomen Dipalmitoylphosphatidylcholin umfassen.

5. Arzneimittel-Freisetzungssystem nach einem vorhergehenden Anspruch, wobei die gasgefüllten Liposomen mit einem Gas, ausgewählt aus der Gruppe, bestehend aus Luft, Stickstoff, Kohlendioxid, Sauerstoff, Argon, Xenon, Helium und Neon, gefüllt sind.

6. Arzneimittel-Freisetzungssystem nach einem vorhergehenden Anspruch, wobei die gasgefüllten Liposomen in einem wässrigen Medium suspendiert gelagert werden.

7. Arzneimittel-Freisetzungssystem nach den Ansprüchen 1 bis 5, wobei die gasgefüllten Liposomen trocken gelagert werden.

8. Arzneimittel-Freisetzungssystem nach einem vorhergehenden Anspruch, wobei die gasgefüllten Liposomen eine Stabilität von mehr als drei Wochen aufweisen.

9. Arzneimittel-Freisetzungssystem nach einem vorhergehenden Anspruch, wobei die gasgefüllten Liposomen ein Reflexionsvermögen von größer als etwa 2 dB haben.

10. Arznelmlttel-Freisetzungssystem nach Anspruch 9, wobei die gasgefüllten Liposomen ein Reflexionsvermögen zwischen 2 dB und 20 dB aufweisen.

11. Verfahren zur Herstellung eines Arzneimittel-Freisetzungssystems, welches die folgenden Schritte umfaßt
(i) das Anlegen von negativem Druck an ein Gefäß, welches Liposomen mit einem Arzneimittel darin eingekapselt enthält,
(ii) das Inkubieren der Liposomen unter dem negativen Druck bei einer Temperatur von oberhalb 0°C für eine Zeitdauer, die ausreichend ist, mindestens 90% der Flüssigkeit von den Liposomen zu entfernen, und
(iii) das Einbringen von Gas in die Liposomen, bis Umgebungsdrücke erreicht werden, wodurch gasgefüllte Liposomen, die das Arzneimittel enthalten, erzeugt werden.

12. Verfahren nach Anspruch 11, welches weiter das Abkühlenlassen der Liposomen vor und während Schritt (i) auf eine Temperatur von zwischen -10°C und -20°C, das Erwärmenlassen der Liposomen während Schritt (ii) auf eine Temperatur von zwischen 10°C und 20°C und das Erwärmenlassen der Liposomen während Schritt (iii) auf Umgebungstemperaturen umfaßt.

13. Verfahren nach Anspruch 11 oder 12, wobei der negative Druck zwischen 700 mm Hg bis 760 mm Hg beträgt und für 24 bis 72 Stunden angelegt wird.

14. Verfahren nach Anspruch 11, 12 oder 13, wobei das Gas in die Liposomen über eine Zeitdauer von 4 bis 8 Stunden eingebracht wird.

15. Verfahren nach einem der Ansprüche 11 bis 14, welches weiter nach Schritt (iii) das Extrudieren der Liposomen durch mindestens einen Filter einer ausgewählten Porengröße umfaßt.

16. Verfahren zur Herstellung von Arzneimittel-Freisetzungssystemen zur Verwendung in Verbindung mit Ultraschallabbildung, welches die folgenden Schritte umfaßt
(i) das Abkühlenlassen der Liposomen mit einem Arzneimittel darin eingekapselt auf eine Temperatur von zwischen -10°C und -20°C,
(ii) das Anordnen der Liposome in ein Gefäß unter einem negativen Druck von zwischen 93,3 kPa (700 mm Hg) bis 101,3 kPa (760 mm Hg),
(iii) das Erwärmenlassen der Liposomen auf eine Temperatur von zwischen 10°C und 20°C,
(iv) das Inkubieren der Liposomen unter dem negativen Druck für 24 bis 72 Stunden, um mindestens 90% der Flüssigkeit aus den Liposomen zu entfemen, und
(v) das Einbringen von Gas in die Liposomen über eine Zeitdauer von 4 bis 8 Stunden, bis Umgebungsdrücke erreicht werden, während zugelassen wird, daß sich die Liposomen auf Umgebungstemperatur erwärmen.

17. Verfahren nach einem der Ansprüche 11 bis 16, wobei das Gas aus der Gruppe, bestehend aus Luft, Stickstoff, Kohlendioxid, Sauerstoff, Argon, Xenon, Neon und Helium, ausgewählt ist.

18. Verfahren nach Anspruch 16, welches weiter nach Schritt (iv) das Extrudieren der Liposomen durch mindestens einen Filter einer ausgewählten Porengröße umfaßt.

19. Verwendung eines Arzneimittel-Freisetzungssystems nach einem der Ansprüche 1 bis 10 zur Herstellung einer Zusammensetzung zur Verwendung bei der Verabreichung eines Arzneimittels einem inneren Körperbereich eines Patienten, wobei die Gegenwart der Liposomen in dem Bereich unter Verwendung von Ultraschall überwacht wird, und das Arzneimittel aus den Liposomen in dem Bereich durch Reißen der Liposomen unter Verwendung von Ultraschall freigesetzt wird.

20. Verwendung nach Anspruch 19, wobei das Arzneimittel dem Linksherz eines Patienten verabreicht wird.

## Revendications

1. Système d'apport de médicament, comprenant des liposomes remplis de gaz, dépourvus de liquide à l'intérieur à au moins 90%, et dans lesquels est encapsulé un médicament, lesdits liposomes étant préparés par un procédé d'instillation de gaz par séchage sous vide comprenant les étapes consistant à :
(i) appliquer une pression négative à un récipient contenant des liposomes dans lesquels est encapsulé ledit médicament ;
(ii) incuber lesdits liposomes à ladite pression négative, à une température supérieure à 0°C, pendant une durée suffisante pour éliminer desdits liposomes au moins 90% du liquide ; et
(iii) instiller un gaz dans lesdits liposomes jusqu'à ce que des pressions ambiantes soient atteintes, pour produire des liposomes remplis de gaz qui contiennent ledit médicament.

2. Système d'apport de médicament, comprenant des liposomes échogènes, remplis de gaz, dépourvus de liquide à l'intérieur à au moins 90%, et dans lesquels est encapsulé un médicament, lesdits liposomes pouvant être amenés à se rompre ou à libérer ledit médicament par application d'ultrasons.

3. Système d'apport de médicament selon la revendication 1 ou la revendication 2, dans lequel lesdits liposomes remplis de gaz sont constitués de matières lipidiques choisies dans le groupe constitué par les acides gras, les lysolipides, la dipalmitoylphosphatidylcholine, la phosphatidylcholine, l'acide phosphatidique, la sphingomyéline, le cholestérol, l'hémisuccinate de cholestérol, l'hémisuccinate de tocophérol, la phosphatidyléthanolamine, le phosphatidylinositol, les lysolipides, les glycosphingolipides, les glucolipides, les glycolipides, les sulfatides, les lipides avec des acides gras à liaison éther et ester, et les lipides polymérisés.

4. Système d'apport de médicament selon la revendication 2, dans lequel lesdits liposomes remplis de gaz sont constitués de dipalmitoylphosphatidylcholine.

5. Système d'apport de médicament selon l'une quelconque des revendications précédentes, dans lequel lesdits liposomes remplis de gaz sont remplis d'un gaz choisi dans le groupe constitué par l'air, l'azote, le dioxyde de carbone, l'oxygène, l'argon, le xénon, l'hélium et le néon.

6. Système d'apport de médicament selon l'une quelconque des revendications précédentes, dans lequel lesdits liposomes remplis de gaz sont conservés en suspension dans un milieu aqueux.

7. Système d'apport de médicament selon les revendications 1 à 5, dans lequel lesdits liposomes remplis de gaz sont conservés à sec.

8. Système d'apport de médicament selon l'une quelconque des revendications précédentes, dans lequel lesdits liposomes remplis de gaz ont une stabilité supérieure à trois semaines.

9. Système d'apport de médicament selon l'une quelconque des revendications précédentes, dans lequel lesdits liposomes remplis de gaz ont un pouvoir réfléchissant supérieur à environ 2 dB.

10. Système d'apport de médicament selon la revendication 9, dans lequel lesdits liposomes remplis de gaz ont un pouvoir réfléchissant compris entre 2 dB et 20 dB.

11. Procédé de préparation d'un système d'apport de médicament comprenant les étapes suivantes :
(i) appliquer une pression négative à un récipient contenant des liposomes dans lesquels est encapsulé un médicament ;
(ii) incuber lesdits liposomes à ladite pression négative, à une température supérieure à 0°C, pendant une durée suffisante pour éliminer desdits liposomes au moins 90% du liquide ; et
(iii) instiller un gaz dans lesdits liposomes jusqu'à ce que des pressions ambiantes soient atteintes, pour produire des liposomes remplis de gaz qui contiennent ledit médicament.

12. Procédé selon la revendication 11, comprenant en outre les étapes qui consistent à laisser refroidir lesdits liposomes avant et durant l'étape (i) à une température comprise entre -10°C et -20°C, à laisser lesdits liposomes se réchauffer durant l'étape (ii) à une température comprise entre 10°C et 20°C et à laisser lesdits liposomes se réchauffer durant l'étape (iii) aux températures ambiantes.

13. Procédé selon la revendication 11 ou 12, dans lequel ladite pression négative est comprise entre 700 mm de Hg et 760 mm de Hg et est appliquée pendant 24 à 72 heures.

14. Procédé selon la revendication 11, 12 ou 13, dans lequel ledit gaz est instillé dans lesdits liposomes sur une période de 4 à 8 heures.

15. Procédé selon l'une quelconque des revendications 11 à 14, comprenant en outre, après l'étape (iii), l'extrusion desdits liposomes à travers au moins un filtre ayant une taille de pores choisie.

16. Procédé de préparation de systèmes d'apport de médicament à utiliser conjointement avec une visualisation par ultrasons, comprenant les étapes suivantes :
(i) faire refroidir les liposomes dans lesquels est encapsulé un médicament à une température comprise entre -10°C et -20°C ;
(ii) placer lesdits liposomes dans un récipient sous pression négative comprise entre 93,3 kPa (700 mm de Hg) et 101,3 kPa (760 mm de Hg) ;
(iii) laisser lesdits liposomes se réchauffer à une température comprise entre 10°C et 20°C ;
(iv) incuber lesdits liposomes sous ladite pression négative pendant 24 à 72 heures pour éliminer desdits liposomes au moins 90% du liquide ; et
(v) instiller un gaz dans lesdits liposomes pendant une période de 4 à 8 heures jusqu'à ce que des pression ambiantes soient atteintes, tout en laissant lesdits liposomes se réchauffer à température ambiante.

17. Procédé selon l'une quelconque des revendications 11 à 16, dans lequel ledit gaz est choisi dans le groupe constitué par l'air, l'azote, le dioxyde de carbone, l'oxygène, l'argon, le xénon, le néon et l'hélium.

18. Procédé selon la revendication 16, comprenant en outre, après l'étape (iv), l'extrusion desdits liposomes à travers au moins un filtre ayant une taille de pores choisie.

19. Utilisation d'un système d'apport de médicament selon l'une quelconque des revendications 1 à 10 dans la préparation d'une composition à utiliser pour apporter un médicament dans une région interne du corps d'un patient, dans laquelle la présence des liposomes dans la région est surveillée en utilisant des ultrasons et le médicament est libéré des liposomes dans ladite région par rupture des liposomes à l'aide d'ultrasons.

20. Utilisation selon la revendication 19, dans laquelle le médicament est apporté au coeur gauche d'un patient.
